# NOUVEAU FASCICULE DE BREVET EUROPEEN

(11) **EP 0 975 307 B2**
(45) Date de publication et mention de la décision concernant l'opposition: **10.11.2010**
(45) Mention de la délivrance du brevet: 15.09.2004
(21) Numéro de dépôt: 98920606.5
(22) Date de dépôt: 10.04.1998
(51) Int. Cl.: A61K 8/14, A61Q 5/02, A61Q 17/00, A61Q 19/00, A01N 25/24, A01N 25/28

(54) **PROCEDE DESTINE A FAIRE ADHERER UN PRODUIT SUR UNE SURFACE**
VERFAHREN ZUR ERHÖHUNG DER HAFTUNG EINES PRODUKTES AUF EINER OBERFLÄCHE
METHOD FOR MAKING A PRODUCT ADHERE TO A SURFACE

(30) Priorité: 14.04.1997 FR 9704548
(43) Date de publication de la demande: 02.02.2000
(73) Titulaire: CAPSULIS, 33600 Pessac (FR)
(72) Inventeur: LAVERSANNE, René, F-33600 Pessac (FR); DEGERT, Corinne, F-33160 Saint Médard en Jalles (FR); ROUX, Didier, F-33700 Mérignac (FR)
(74) Mandataire: Lecca, Patricia S.
(86) Numéro de dépôt international: PCT/FR1998/000729
(87) Numéro de publication internationale: WO 1998/046199

(56) Documents cités:
- EP-A- 0 457 910
- EP-A- 0 459 856
- EP-A1- 0 457 910
- WO-A-90/06747
- WO-A-92/19214
- WO-A-93/19735
- WO-A-95/23578
- WO-A-96/31196
- WO-A1-96/26179
- WO-A2-92/19214
- US-A- 4 855 090
- US-A- 5 264 618
- PATENT ABSTRACTS OF JAPAN vol. 009, no. 074 (C-273), 3 avril 1985 & JP 59 210013 A (AJINOMOTO KK), 28 novembre 1984,
- 'Cosmetics & Toiletries', vol. 103, Mars 1988, ALLURED PUBLISHING CORP. article FOX, CHARLES: 'An introduction to the formulation of shampoos', pages 25 - 58

## Description

La présente invention concerne un procédé destiné à faire adhérer un produit sur une surface.

Elle concerne également les applications d'un tel procédé dans différents domaines dépendant de la nature de la surface et de celle du produit.

Elle s'applique tout particulièrement au traitement de surfaces biologiques, telles que la peau ou les cheveux de l'être humain ou la peau ou le pelage des animaux ou la cuticule des plantes ou des insectes.

Elle s'applique également au traitement des fibres, aussi bien des fibres naturelles qu'artificielles, ainsi que des produits à base de fibres tels que les tissus.

On connaît différents domaines de l'industrie où l'on cherche à améliorer l'interaction entre un produit et une surface ou à fixer un produit sur une surface soit pour améliorer l'état de cette surface, soit pour lui faire subir un traitement particulier. Il peut s'agir, en particulier, d'applications où l'on cherche à modifier les propriétés d'une surface en lui faisant subir un traitement particulier par un produit que l'on souhaite voir rester suffisamment longtemps au contact de cette surface.

Il est souvent utile de pouvoir traiter les fibres ou les surfaces textiles ou naturelles pour améliorer leur apparence (brillance, couleur, parfum...), leurs propriétés (solidité, élasticité, glissage...), pour leur apporter de nouvelles fonctionnalités.

Dans le cas de la peau, des poils et cheveux, on cherche souvent à leur apporter des actifs traitant, soignant ou fortifiant, par exemple.

D'une façon générale, on désigne ci-après indifféremment par "actif" ou "produit actif' le produit que l'on désire fixer sur une surface.

L'un des problèmes rencontrés pour ces différents traitements est d'assurer la rémanence du produit sur la surface, qu'il s'agisse d'une surface inerte ou d'une surface biologique telle que la peau, les poils, les cheveux ou les phanères des êtres vivants ou la surface ou cuticule des plantes. Bien souvent, le traitement est appliqué par réaction chimique ou par adsorption physico-chimique (coloration par exemple) lors de la fabrication de la fibre, ou dans le cas des cheveux par un traitement demandant une application spéciale.

Si l'on essaie d'appliquer le traitement lors d'une opération de lavage ou lors d'un shampooing par exemple, le problème principal rencontré est le fait que l'actif apporté par le shampooing ou le produit lessiviel qui est rincé, se trouve perdu pour sa très grande majorité, seulement une infime partie restant fixée sur la surface traitée. La rémanence est donc faible, même si la proportion d'actif dans le produit est forte. Le même type de problème peut se poser si la surface traitée se trouve ensuite exposée à l'action de la pluie par exemple.

La microencapsulation est une solution souvent utilisée pour prolonger la durée de mise à disposition d'un actif en ne le libérant que lentement. Elle est la plupart du temps réalisée par la formation d'une coque polymère autour du principe actif, soit par pulvérisation de l'actif en présence d'un polymère, soit par l'une des nombreuses méthodes d'encapsulation développées industriellement, comme les différentes techniques de coacervation, d'atomisation, de coprécipitation. Les liposomes peuvent aussi être utilisés, mais leur faible stabilité et leur coût empêchent de les utiliser industriellement.

La demande internationale WO 95/23578 décrit l'utilisation de liposomes cationiques pour déposer un ingrédient actif sur les cheveux. Le principal problème posé par de tels liposomes est que la technique utilisée pour leur préparation qui est faite par simple addition des composants de la membrane liposomale dans la solution d'actif ne permet que difficilement de contrôler le taux d'encapsulation qui, en tout état de cause, reste faible. Par ailleurs, seuls des actifs insolubles dans l'eau peuvent être encapsulés dans de tels liposomes, ce qui limite considérablement la liste des produits actifs utilisables dans une telle technique.

On a maintenant découvert que des microcapsules multilamellaires à structure en oignon, ci-après également désignées par microvésicules ou vésicules multilamellaires, à condition d'être formulées pour présenter une charge globale positive, présentaient la propriété de se fixer de façon particulièrement stable sur différentes surfaces et plus particulièrement sur la surface de fibres naturelles ou synthétiques ou des produits à base de telles fibres ou sur les cheveux ou les poils.

Par vésicules multilamellaires à structure en "oignon", on entend des vésicules multilamellaires de forme sensiblement sphérique, constituées d'une succession de bicouches concentriques et, cela, du centre à la périphérie des vésicules, d'où le nom de structure en oignon utilisé par analogie, pour qualifier de telles structures.

Ces structures peuvent être mises en évidence par examen microscopique des compositions. L'observation se fait en utilisant un microscope optique en lumière polarisée, dans lequel une phase lamellaire, biréfringente est visible. Elle se manifeste par une texture caractéristique, liée à la présence des défauts (joints de grains) entre les domaines de phase orientés différemment. Dans le cas de la phase concentrée de vésicules, la texture est caractérisée par son caractère uniforme et fin, relié à la taille des vésicules. Dans la phase dispersée de vésicules, celles-ci sont visibles sous la forme de points plus ou moins résolus (en fonction de la taille), légèrement biréfringents. La biréfringence ne s'observe que lorsque la dispersion n'est pas trop diluée. Il y aura donc lieu, si la dispersion est relativement diluée de procéder à une opération préalable de concentration pour mettre clairement en évidence la biréfringence caractéristique de la présence des vésicules à structure en oignon.

Il est bien évident que dans le cas présent, comme dans le cas des liposomes cationiques décrits dans la demande internationale WO 95/23578, la charge cationique de l'entité liposome ou microcapsule se trouve compensée à l'équilibre par des contre-ions. Toutefois, cette charge peut être mise en évidence par des mesures de potentiels zhéta réalisées au moyen d'un zhétamètre. De telles expériences sont basées sur des mesures de mobilité en présence d'un champ électrique.

Les vésicules utilisées selon l'invention peuvent être obtenues de façon particulièrement simple par formation d'une phase cristal-liquide lamellaire et en provoquant le réarrangement des bicouches formées pour former des vésicules multilamellaires. Un procédé de ce type permettant de faire des microcapsules de taille contrôlée est décrit dans la demande internationale WO 93/19735 qui décrit un procédé qui permet, grâce au recours à une étape de cisaillement homogène d'une phase cristal-liquide lamellaire, de préparer des microcapsules de taille contrôlée, non seulement à partir de tensioactifs lipidiques susceptibles de former des liposomes mais aussi à partir des différents tensioactifs anioniques ou non ioniques et propose l'encapsulation de substances notamment biologiques dans ces capsules.

La demande internationale WO 95/19707 décrit, quant à elle, un procédé destiné à améliorer la rémanence d'une odeur consistant à encapsuler un principe actif odoriférant au sein d'une microcapsule constituée d'un arrangement multilamellaire de bicouches concentriques séparées par un milieu aqueux. Ces microcapsules sont obtenues en préparant une phase cristal-liquide ou une suspension de phase cristal-liquide à partir d'au moins un agent tensioactif et en provoquant le réarrangement des bicouches sous forme de microcapsules. Ce réarrangement peut être, en particulier, obtenu en utilisant le procédé décrit dans la demande internationale WO 93/19735 citée précédemment.

Selon l'invention, le produit actif que l'on souhaite faire adhérer à une surface à partir d'une composition est incorporé en quasi totalité à l'intérieur de vésicules multilamellaires que l'on désignera dans le présent mémoire indifféremment par microcapsules, microvésicules ou vésicules. Ces microcapsules sont avantageusement de forme sensiblement sphérique et sont constituées de lamelles concentriques leur conférant une structure de type "oignon".

La substance active se trouve ainsi incluse au sein même de la microcapsule, généralement dans ses membranes, le cas échéant si elle est purement hydrophile, dans l'eau ou le liquide interstitiel inclus à l'intérieur de la microcapsule. Mais elle fait toujours partie intégrante de la microcapsule.

Même si, d'une façon générale, on utilise des milieux eau/tensioactif(s) pour réaliser les microcapsules de l'invention, il n'est nullement exclu de remplacer l'eau par un solvant polaire, par exemple le glycérol.

Selon un autre avantage, la technologie proposée selon l'invention permet la préparation de vésicules ayant un très fort rendement d'encapsulation, notamment un rendement supérieur à 90 % et généralement très proche de 100 %. D'un emploi facile, cette technologie permet également la préparation de grandes quantités de produits encapsulés. De plus, elle ne recourt pas à un cosolvant organique, ce qui permet d'envisager toutes sortes d'applications industrielles, en particulier dans des domaines où l'utilisation de solvants organiques est prohibée. Ceci représente un avantage tout particulier dans l'industrie cosmétique, pharmaceutique ou alimentaire où l'on cherche à éviter, dans la mesure du possible, l'utilisation de solvants organiques souvent difficilement éliminables complètement. Mais ceci est également intéressant dans d'autres domaines de l'industrie où la tendance actuelle est de remplacer les solvants organiques par des milieux aqueux.

Un autre avantage vient du fait que l'utilisation des tensioactifs confère une bonne dispersabilité à la formulation qui peut être utilisée sous forme liquide, en dispersion aqueuse. Cet aspect est particulièrement avantageux lorsque l'on a affaire à des molécules hydrophobes ou non solubles dans l'eau qui peuvent être dispersées grâce à l'invention sans avoir recours à un solvant organique.

Par agent cationique, on entend un produit portant une charge positive.

Selon l'une de ses caractéristiques essentielles, l'invention concerne un procédé destiné à faire adhérer un produit sur une surface, caractérisé en ce qu'il consiste à mettre en contact avec ladite surface une composition dans laquelle ledit produit est incorporé dans des vésicules multilamellaires sensiblement sphériques, de diamètre compris entre 0,1 et 100 µm, constituées de membranes concentriques à base d'au moins un tensioactif séparées par un milieu solvant, lesdites vésicules présentant une structure en oignon et portant une charge globale positive liée à la présence d'au moins un agent cationique au sein desdites vésicules, ledit agent cationique étant un polymère cationique encapsulé dans lesdites vésicules multilamellaires.

Les vésicules multilamellaires dont l'utilisation est revendiquée dans la présente demande permettent d'encapsuler avec un très bon rendement un nombre important d'actifs. De plus, la composition du mélange de tensioactifs composant les membranes des vésicules peut être adaptée à l'application envisagée et ces vésicules peuvent être préparées à partir de toutes les classes de tensioactifs.

L'invention s'applique tout particulièrement à la fixation d'un produit sur une surface présentant une charge négative, ce qui est le cas de la plupart des surfaces naturelles ainsi que d'un nombre important de surfaces artificielles, en particulier des fibres, des poils, des cheveux, de la peau, des phanères et de la cuticule des plantes et des insectes.

Selon l'un de ses aspects essentiels, l'invention concerne le traitement, en vue de fixer un actif, d'une fibre naturelle ou artificielle ou d'un ensemble de fibres tel qu'un tissu.

Selon un autre aspect particulièrement important de l'invention, la surface à traiter sera une surface biologique, en particulier une partie externe du corps humain ou animal telle que la peau, les phanères, les poils, les cheveux ou la cuticule ou les pilosités des insectes ou d'une partie aérienne des végétaux, en particulier leur cuticule.

Il pourra s'agir, selon une variante particulière, d'un traitement cosmétique ou d'hygiène du corps humain ou animal destiné notamment à augmenter la rémanence d'un agent actif sur la partie du corps traité, par application d'une composition cosmétique ou d'hygiène dans laquelle ledit produit actif est incorporé dans des vésicules multilamellaires sensiblement sphériques, de diamètre compris entre 0,1 et 100 µm, constituées de membranes concentriques à base d'au moins un tensioactif séparées par un milieu solvant, lesdites vésicules présentant une structure en oignon dans lesquelles est encapsulé un polymère cationique et portant une charge globale positive.

Il est bien entendu que la nature du produit à fixer dépendra étroitement de la surface à traiter et du résultat visé. Toutefois, et cela constitue un des avantages essentiels de l'invention, les microcapsules utilisées selon l'invention pour amener le produit en contact avec la surface permettent, du fait de leur nature et de leur procédé d'obtention, d'y incorporer un nombre quasiment illimité de produits dont on veut assurer la fixation sur la surface à traiter. Ceci constitue, par ailleurs, un gros avantage de l'invention, en particulier par rapport aux techniques mettant en oeuvre des liposomes. Il est en effet bien connu de l'homme du métier que les liposomes, du fait de la technologie de fabrication, ne peuvent contenir qu'un nombre limité de produits encapsulés.

Les vésicules utilisées selon l'invention doivent présenter une charge globale positive.

Une telle charge est conférée en encapsulant à l'intérieur de la microcapsule un polymère cationique.

A titre d'agents cationiques encapsulés pour conférer une charge positive à la vésicule, on utilise un polymère cationique.

A titre d'exemples de tels polymères, on citera :
- les dérivés de polysaccharides, d'origine naturelle, biotechnologique ou synthétique, naturellement cationiques ou quaternisés,
- les hydrolysats cationiques de protéines,
- Les dérivés polyaminés, éventuellement substitués par des chaînons polyéthylèneglycol,
- les polyaminoacides dans les conditions de pH où ils sont cationiques,
- la polyéthylèneimine,
- les dérivés quatemisés de polyvinylpyrrolidone (PVP) et les copolymères de polyvinylpyrrolidone quatemisée et de polymères hydrophiles (uréthane, acrylate, etc.),
- les polyquatemium, qui sont des polymères cationiques décrits dans International Cosmetic Ingredient Dictionary publié par la CTFA (Cosmetic, Toiletry and Fragrance Association),
- les dérivés de chitine.

Il est apparu qu'une faible quantité de polymère cationique; est suffisante pour assurer l'adhésion des vésicules sur la surface. Plus précisément, les vésicules multilamellaires de l'invention contiennent avantageusement de 0,01 % à 10 %, de préférence de 0,1 % à 5 % en poids par rapport au poids total de la composition de la vésicule d'un tel agent cationique.

Comme on l'a vu précédemment, le choix du produit à fixer dépend étroitement de la surface à traiter. Il n'est pas exclu que, dans certains cas, le produit à fixer soit constitué par le polymère cationique encapsulé.

C'est le cas en particulier de l'utilisation de polymères cationiques lorsque l'on veut gainer un cheveu ou un poil, comme cela est bien connu dans le domaine de la cosmétique ou de l'hygiène. Dans ce cas, le produit à fixer pourra être le polymère lui-même qui jouera à la fois la fonction d'agent cationique conférant une charge positive globale à la vésicule multilamellaire et de produit à fixer.

Le produit actif peut être également constitué d'un tensioactif cationique, par exemple un ammonium quaternaire qui pourra être utilisé à la fois comme bactéricide pour traiter une surface et comme constituant des membranes des vésicules.

Le champ des traitements visés par l'invention s'étend également aux traitements pharmaceutiques ou vétérinaires topiques de la peau ou de la chevelure des êtres humains ainsi que de la peau ou du pelage des animaux.

L'invention vise donc également des procédés où l'on cherche à fixer sur le corps humain ou animal une composition pharmaceutique comprenant un agent actif incorporé dans un véhicule pharmaceutiquement acceptable. Elle concerne en particulier une composition pharmaceutique destinée au traitement du corps humain ou animal dans laquelle au moins un agent actif fait partie intégrante de microvésicules telles qu'elles ont été décrites précédemment.

Comme on l'a vu précédemment, un des avantages de la présente invention est de fournir une technique particulièrement souple permettant de préparer des compositions incorporant un produit que l'on souhaite fixer sur une surface.

En effet, les compositions destinées à la mise en oeuvre du procédé décrit précédemment ou les compositions pharmaceutiques telles que décrites ci-dessus sont aisément préparées à partir d'une phase lamellaire cristal-liquide à base d'au moins un agent tensioactif, comprenant un agent cationique et, le cas échéant, un produit à fixer ou un agent différent dudit agent cationique, en provoquant un cisaillement de ladite phase lamellaire cristal-liquide conduisant à la formation desdites vésicules puis en incorporant lesdites vésicules dans un milieu adapté ledit agent cationique étant un polymère cationique encapsulé dans lesdites vésicules multilamellaires.

Ce milieu sera bien entendu choisi en fonction de l'application visée pour la composition contenant les vésicules cationiques. Il pourra, en particulier, s'agir d'un milieu aqueux, huileux ou organique. Dans chaque cas, bien entendu, la formulation des vésicules multilamellaires sera adaptée en fonction de la nature du milieu de dispersion.

Ainsi, par exemple, dans le domaine de la cosmétique ou de l'hygiène humaine ou animale, les compositions incorporant les vésicules seront avantageusement sous forme de lotions aqueuses, de shampooings, d'émulsions, de gels, de dispersions huileuses, de baumes, de solutions pour aérosol ou de formulations pour applications transdermiques.

Les vésicules multilamellaires pourront être dispersées dans un milieu aqueux.

Les vésicules multilamellaires cationiques pourront également être dispersées dans un milieu lipophile, par exemple une huile ou un mélange de cire et d'huile. De tels milieux pourront s'avérer particulièrement intéressants, en particulier dans tous les domaines d'application où l'on cherche à faire adhérer un principe actif sur la peau, les cheveux ou les poils des êtres humains ou des animaux. A titre d'exemples de tels domaines, on citera en particulier celui des produits cosmétiques, par exemple des produits destinés au maquillage tels que les mascaras et les rouges à lèvres ou celui des déodorants corporels non alcooliques comprenant comme excipient un gel stéarique ou une huile de silicone.

Un autre domaine d'application où l'on pourra faire appel avantageusement à des vésicules cationiques dispersées dans un milieu lipophile est celui des traitements appliqués de façon topique sur la peau des animaux. De tels traitements sont couramment désignés sous le vocable anglais "pour on" et consistent à répandre sur le pelage des animaux une composition à effet topique.

Le polymère cationique est introduit dans la composition de la phase lamellaire, avant de provoquer le cisaillement qui conduit à la formation des vésicules. De ce fait, il est coencapsulé avec l'actif, à l'intérieur de la vésicule, et en fait partie intégrante. La formulation des tensioactifs est adaptée afin de tenir compte de la présence du polymère cationique.

La nature des produits actifs utilisables en tant que produits à fixer sur une surface dépend, bien entendu, du type de surface et du traitement visé.

Les actifs utilisables sont, par exemple, ceux utilisés en cosmétique, pour le traitement des cheveux, dans les traitements d'hygiène du corps humain ou animal, mais également ceux utilisés en pharmacie humaine ou vétérinaire, pour des applications à usage topique, ainsi que ceux utilisés dans le domaine de l'industrie textile.

On peut citer par exemple dans le domaine de la cosmétique et de la pharmacie humaine et vétérinaire :
- des actifs essentiellement hydrosolubles tels que, par exemple, extraits de plantes, d'algues, vitamines, protéines hydrosolubles, hydrolysat de protéines, peptides, α-hydroxyacides, acide salicylique, dérivés de la caféine, des produits hydratants tels que le glycérol ou les dérivés du glycol
- des actifs essentiellement liposolubles tels que, par exemple, huiles végétales et animales, huiles synthétiques, carbonées ou siliconées, huiles essentielles et leurs mélanges, parfums et arômes, vitamines, dérivés d'acides gras.

D'autres produits actifs peuvent également être avantageusement encapsulés en vue d'améliorer leur adhésion envers une surface à traiter, on citera en particulier :
- les bactéricides, comme les dérivés du triclosan, les ammoniums quaternaires, les dérivés du zinc (zinc-pyrithione, undécylénate de zinc), la piroctone olamine, etc.,

L'invention s'applique également tout particulièrement à toutes les applications où l'on cherche à améliorer la fixation d'un insecticide sur une cible. On pourra donc citer également à titre de produits actifs à fixer, les insecticides, en particulier :
- pyréthrine et pyréthrinoïdes de synthèses (perméthrine et dérivés),
- composés phosphorés, par exemple malathion, parathion,
- composés organochlorés, par exemple lindane.

Parmi les cibles de choix visées en tant que surface dans le cadre du procédé de la présente invention, on citera tout particulièrement les plantes et les insectes.

Les plantes et les insectes sont entourés d'une membrane analogue à la peau appelée cuticule, même si elle est de nature différente entre les insectes et les plantes. La cuticule des insectes est une couche dure de kératine (analogue à la kératine des cheveux). Elle est en général recouverte, au moins partiellement, de pilosités à effet tactile, sensoriel ou moteur, appelées phanères, de nature chimique analogue à la cuticule. Ce sont donc des surfaces de choix pour l'adhésion des vésicules de l'invention. La fixation de telles vésicules directement sur la cuticule ou les pilosités de l'insecte est de nature à renforcer l'action des insecticides, en en prolongeant la durée de contact avec l'insecte. Ceci est particulièrement important dans le cas de traitements sur l'homme ou l'animal, tels que les traitements antiparasitaires, anti-acariens, antipoux, dans lesquels on cherche, d'une part à réduire la dose d'insecticide employé et où, d'autre part, les produits sont rarement associés à des shampooings pour éviter leur élimination lors du rinçage.

En ce qui concerne les plantes, les mêmes mécanismes d'adhésion peuvent être mis en oeuvre. L'utilité d'une telle adhésion est là encore évidente, car dans le cas du traitement des plantes, le rinçage est présent de manière naturelle sous l'effet de la pluie. Il est donc très important de retenir au niveau de la cuticule un maximum de matière active pour obtenir l'action en surface recherchée en particulier dans le cas des insecticides et fongicides de surface.

La technique de préparation consiste à préparer, dans une première étape, une phase lamellaire cristal-liquide contenant un mélange du tensioactif ou des tensioactifs, du polymère cationique, du solvant polaire, de l'eau de préférence, et le cas échéant de produit ou mélange actif que l'on cherche à encapsuler, puis à provoquer par un cisaillement la formation des vésicules multilamellaires.

Pour optimiser le rendement d'encapsulation, on choisira des conditions telles que la phase cristal-liquide soit homogène, c'est-à-dire monophasique, de façon à ce que l'ensemble du solvant polaire (eau en général), du produit ou mélange actif soit solubilisé dans cette phase lamellaire.

Les conditions optimales à utiliser pourront être en général déterminées par examen d'une série de compositions contenant des quantités variables de solvant et de produit actif. Cet examen sera fait soit par observation macroscopique en observant la séparation de phase, soit par observation microscopique en utilisant un microscope optique, de préférence un microscope polarisant.

Toutefois, la formation d'une phase cristal-liquide n'est pas une condition suffisante pour obtenir, par la suite, une suspension aisément dispersable de vésicules multilamellaires. Il faut, de plus, obtenir une organisation de cette phase lamellaire sous la forme d'un empilement compact de ces vésicules. Ce réarrangement pourra être obtenu en appliquant un cisaillement homogène, comme décrit dans la demande de brevet WO 93/19735. Ce réarrangement pourra être également obtenu en jouant sur la formulation particulière du mélange, en particulier en choisissant un mélange de tensioactifs tels que la texture recherchée, sous forme de vésicules multilamellaires, se forme spontanément ou à défaut lors d'une simple sollicitation mécanique, par exemple lors du mélange des produits qui provoque une telle sollicitation mécanique.

C'est pourquoi, on choisira avantageusement un mélange de tensioactifs et des concentrations respectives de chacun des tensioactifs contenus dans ce mélange de façon à obtenir la texture désirée.

Plus précisément, on utilisera un mélange de tensioactifs constitué généralement de deux types de tensioactifs, l'un étant plutôt soluble dans l'eau et présentant donc une HLB élevée et l'autre étant plutôt soluble dans l'huile et présentant donc une HLB relativement basse. Par ailleurs, il sera particulièrement avantageux que l'un au moins des tensioactifs ait une CMC relativement basse, de préférence inférieure à 10⁻⁵ mole/litre, de préférence encore inférieure à 10⁻⁶ mole/l.

La proportion en poids des tensioactifs dans le mélange final se situe généralement entre 5 et 90 %, de préférence entre 30 et 70 %.

Plus précisément, pour obtenir les microcapsules recherchées, on utilisera des mélanges de départ présentant les propriétés suivantes :
1) Le mélange doit former une phase lamellaire cristal-liquide homogène pour des proportions d'eau, en poids, allant de 10 à 98 %, plus généralement de 20 à 60 %.
2) Cette phase lamellaire homogène doit présenter une texture spécifique, c'est-à-dire un arrangement spatial des lamelles qui, soit spontanément, soit sous simple mélange, soit encore sous l'action d'un cisaillement spécifique tel que décrit dans la demande internationale WO 93/19735, corresponde à une structure en "oignon". Cette structure peut être reconnue aisément par l'homme de métier en utilisant un microscope polarisant.

Pour obtenir les deux conditions ci-dessus, on utilisera avantageusement, comme on l'a expliqué précédemment, deux tensioactifs ayant des équilibres hydrophile/lipophile sensiblement différents, afin de pouvoir ainsi régler à loisir les propriétés d'organisation (texture) de la phase lamellaire.

On choisira de préférence de mélanger un tensioactif plutôt lipophile présentant une HLB basse comprise entre 3 et 7 et un tensioactif hydrophile présentant une HLB élevée comprise entre 8 et 15. L'homme du métier pourra aisément, en faisant varier les proportions des deux types de tensioactifs, obtenir une phase lamellaire homogène ayant des propriétés de texture recherchées.

Les deux types de tensioactifs seront choisis parmi les tensioactifs compatibles avec l'usage visé.

En appliquant le procédé de préparation précédemment décrit, on aboutit, selon la formulation de la phase lamellaire cristal-liquide, et plus précisément en fonction de la nature et/ou des proportions des tensioactifs mis en oeuvre, à deux types de vésicules différentes entre elles par le degré d'organisation des molécules tensioactives dans la membrane constituant les compartiments des vésicules multilamellaires :
- les vésicules de type "fluide" correspondent à des membranes où les molécules de tensioactifs sont libres de se mouvoir et ne sont pas organisées sous forme d'un réseau cristallin bidimensionnel. Elles ont, en général, une forme sphérique.
- les vésicules de type "solide" correspondent, au contraire à une organisation des molécules de tensioactif sous la forme d'un réseau cristallin bidimensionnel. La forme de ces vésicules est anisotrope et se présente le plus souvent sous la forme de petits cristaux facettés. Dans tous les cas, la taille des vésicules est comprise en 0,1 et 100 µm. L'aspect facetté de ces vésicules n'est pas contradictoire avec leur structure multilamellaire de type oignon.

### Exemples

### Exemple 1 : Shampooing

Des vésicules multilamellaires de tensioactif sont préparées à partir de la formulation suivante :

| | |
|---|---|
| sorbitan stéarate | 25 % |
| polysorbate 60 | 20 % |
| Jaguar C13S | 5 % |
| Solution aqueuse d'actifs hydrosolubles | 50 %. |

Parmi les actifs solubles dans l'eau, on peut utiliser les α-hydroxyacides, l'acide salicylique, la vitamine C, la caféine, les protéines (entières ou hydrolysées), les peptides, etc. Le jaguar C 13S, de la société Rhône-Poulenc est un dérivé quaternisé de la farine de guar.

Les constituants sont mélangés à 50°C puis refroidis sous agitation constante par agitation mécanique puis dispersés dans une base de shampooing formée de 15 % de lauryléthersulfate de sodium dans l'eau, à raison de 3 % de vésicules dans la base shampooing. La suspension est homogène et laiteuse, à cause de la présence des vésicules qui diffusent la lumière.

Pour les essais d'adhésion, une touffe de cheveux est trempée dans la solution de shampooing contenant les microcapsules, puis rincée à l'eau courante, puis séchée à l'air. L'observation est effectuée sur les poils séchés, par microscopie électronique à balayage, sous vide de 10⁻⁷ Torr, après métallisation de l'échantillon.

Les clichés réalisés avec un grossissement de 2 500 montrent clairement la présence des vésicules collées aux cheveux, avec une répartition uniforme sur la surface du cheveu.

Par ailleurs, les clichés réalisés avec un grossissement de 6 000 montrent clairement que les vésicules adhèrent fortement à la surface du cheveu, qu'elles "mouillent" littéralement.

### Exemple 2 : Composition antipoux

Des microvésicules multilamellaires encapsulant le malathion comme insecticide sont préparées à partir de la formulation suivante :

| | |
|---|---|
| Polysorbate 60 | 25 g |
| Stéarate de sorbitan | 32 g |
| Jaguar C13S | 3 g |
| Malathion | 10 g |
| Eau tamponnée (pH=6) | 30 g |

Les vésicules sont obtenues par mélange (erlenmeyer, agitation mécanique) des tensioactifs et de l'eau à température ambiante, puis en maintenant l'agitation, chauffage à 60°C. Quand le mélange est homogène le chauffage est arrêté, et l'agitation maintenue, puis dès que la température est inférieure à 45°C, le malathion est additionné et le mélange refroidi sous agitation.

On obtient une pâte homogène formée d'un empilement compact de microvésicules lamellaires, qu'on peut identifier par observation de la texture caractéristique en microscopie optique en lumière polarisée.

Cette pâte est dispersée par addition lente d'eau tamponnée, à température ambiante, sous agitation. Le mélange final contient 5% de vésicules, soit 0,5 % de malathion. Sa viscosité peut être ajustée par l'addition d'un viscosant, par exemple le Jaguar C13S ou le jaguar C 162 (de même nature chimique que le C13S). Une proportion de viscosant de 0,2% est suffisante pour donner un produit facile à appliquer.

Cette formulation présente deux avantages. D'une part, elle permet de mettre en oeuvre du malathion dans une base aqueuse, tout en conservant la stabilité du malathion, qui n'est pas connu pour être stable en milieu aqueux. D'autre part elle permet l'accrochage des vésicules sur les cheveux et sur les phanères des insectes, conférant ainsi une longue durée d'action au produit, même après rinçage.

Cet aspect accrochage peut être visualisé par microscopie électronique à balayage, sous vide de 10⁻⁷ Torr, après légère métallisation, sur un échantillon de cheveux infestés de poux, traités avec le produit (application par massage du cuir chevelu 5 min, puis rinçage à l'eau). Les vésicules sont visibles à la fois sur le cheveux et sur les insectes.

### Exemple 3 : Fixation sur une fibre textile

Des microvésicules multilamellaires sont préparées à partir de la formulation suivante :

| | |
|---|---|
| Polysorbate 60 | 20 g |
| Stéarate de sorbitan | 25 g |
| Jaguar C 13S | 5 g |
| Conservateur | 0,8 g |
| Eau | 49,2 g |

Les vésicules sont obtenues par mélange (erlenmeyer, agitation mécanique) des tensioactifs et de l'eau à température ambiante, puis en maintenant l'agitation, chauffage à 60°C. Quand le mélange est homogène, le chauffage est arrêté, et l'agitation étant maintenue, le mélange est refroidi sous agitation.

On obtient une pâte homogène formée d'un empilement compact de microvésicules lamellaires, qu'on peut identifier par observation de la texture caractéristique en microscopie optique en lumière polarisée.

Cette pâte est dispersée par addition lente d'eau, à température ambiante, sous agitation. Le mélange final contient 5% de vésicules. Sa viscosité peut être ajustée par l'addition d'un viscosant, par exemple le Jaguar C13S, à la concentration maximum de 2%.

La très bonne adhésion des vésicules sur les fibres textiles peut être visualisée par microscopie électronique à balayage, sous vide de 10⁻⁷ Torr, après légère métallisation, sur des échantillons de fibres textiles de différentes natures traitées par trempage de la fibre dans la dispersion de vésicules puis rincées à l'eau.

Les vésicules sont visibles sur les fibres que ce soit du polyamide, du polyester ou du coton.

### Exemple 4 : Fixation sur un tissu

Des microvésicules multilamellaires encapsulant un parfum sont préparées à partir de la formulation suivante :

| | |
|---|---|
| Polysorbate 60 | 22 g |
| Stéarate de sorbitan | 25 g |
| Jaguar C 13 S | 3 g |
| Parfum "Floral Sweet"* | 10% |
| Conservateur | 0,8 g |
| Eau | 39,2 g |

| | |
|---|---|
| * parfum fourni par Haarman & Reimer | |

Les vésicules sont obtenues par mélange (erlenmeyer, agitation mécanique) des tensioactifs et de l'eau à température ambiante, puis en maintenant l'agitation, chauffage à 60°C. Quand le mélange est homogène le chauffage est arrêté, le parfum est additionné dès que la température est inférieure à 45°C, puis, l'agitation étant maintenue, le mélange est refroidi sous agitation.

On obtient une pâte homogène formée d'un empilement compact de microvésicules lamellaires, qu'on peut identifier par observation de la texture caractéristique en microscopie optique en lumière polarisée.

Cette pâte est dispersée par addition lente d'eau, à température ambiante, sous agitation. Le mélange final contient 10% de vésicules. Sa viscosité peut être ajustée par l'addition d'un viscosant, par exemple le Jaguar C13S, à la concentration maximum de 0,5%.

Cette dispersion de vésicules "parfumées" peut être utilisée pour parfumer de façon durable des tissus, par accrochage des vésicules sur la fibre textile, et relargage lent du parfum. La pulvérisation d'une petite quantité de cette dispersion sur un tissu confère un parfum qui persiste plusieurs semaines.

### Exemple 5 : déodorant corporel non alcoolique

Les déodorants corporels en vaporisateur sont des solutions alcooliques de parfum et de bactéricide. Si l'on veut éviter l'utilisation d'alcool, essentiellement pour éviter la sensation irritante, on utilise des dispersions dans une huile de silicone légère, qui donne une sensation de fraîcheur lors de son évaporation après application, analogue à celle obtenue avec l'alcool.

Malheureusement tous les actifs utilisés dans les déodorants ne sont pas solubles dans ces huiles de silicone. D'autre part, l'encapsulation permet d'obtenir un effet longue durée du parfum, ce qui améliore l'efficacité du déodorant. Pour ce type d'application il faut formuler des vésicules dispersibles en milieu huile de silicone.

Des vésicules multilamellaires selon l'invention sont préparées à partir de la formulation suivante :

| | |
|---|---|
| Lécithine de soja à 20% de phosphatidylcholine | 40 g |
| Alcool laurique éthoxylé, à 4 oxyde d'éthylène | 10 g |
| Parfum | 18 g |
| Bactéricide (Irgasan DP300 de CIBA) | 3 g |
| Jaguar C13S | 1 g |
| Eau | 28 g |

La lécithine, le polymère cationique, le bactéricide et 50% de l'eau sont préalablement mélangés à température ambiante pour obtenir une pâte homogène. Lorsque le mélange est homogène, l'alcool laurique éthoxylé, le parfum, le reste d'eau sont additionnés. Lorsque l'addition est complète, le mélange est maintenu sous agitation à température ambiante jusqu'à obtention d'une pâte visqueuse, que l'on peut disperser facilement dans une base pour atomiseur déodorant non alcoolique.

Cette base est essentiellement composée d'une huile de silicone légère, dans laquelle peuvent être additionnés des sels d'aluminium (effet bloquant de la transpiration). Les vésicules sont dispersées à 10%, de manière à avoir un titre en bactéricide de 0,3% dans la dispersion finale. Cette dispersion non aqueuse a une tendance à sédimenter du fait de la moins bonne efficacité de l'agitation brownienne en milieu huileux, et nécessite une agitation manuelle pour remettre en suspension les vésicules avant l'utilisation.

### Exemple 6 : Formulation dermatologique vétérinaire

Le traitement antiparasitaire du bétail se fait souvent par utilisation de dispersion de matière active dans un milieu huileux qui est versé en une seule application sur le dos de l'animal. Le principe actif diffuse ensuite par capillarité sur l'ensemble de l'animal. Ce procédé communément appelé "pour on" (qui signifie en anglais "verser sur") présente l'avantage d'être rapide d'application et d'éviter l'inhalation du principe actif, consécutive d'une pulvérisation, à la fois pour l'animal et pour l'applicateur.

Les vésicules selon l'invention se prêtent particulièrement bien à la mise en dispersion dans une huile d'un principe actif, et donc à la formulation de formes "pour on" de principes actifs vétérinaires.

Les vésicules sont préparées selon la composition suivante :

| | |
|---|---|
| Lécithine de soja à 20% de phosphatidylcholine | 40 g |
| Oléate de sorbitan | 10 g |
| Jaguar C13 S | 3 g |
| Principe actif (par exemple perméthrine) | 5 g |
| Eau | 22 g |
| Huile minérale. | 20 g |

Le mélange des constituants se fait à température ambiante, en introduisant les constituants dans l'ordre suivant : lécithine, polymère cationique, oléate de sorbitan, principe actif, eau, puis huile minérale. On obtient une pâte homogène.

Cette pâte peut facilement être dispersée dans une huile minérale, à 10% pour obtenir une dispersion de vésicules encapsulant l'actif, de viscosité adaptée à l'utilisation par dépôt sur le dos de l'animal. Comme pour l'exemple 7, la dispersion nécessite une agitation manuelle avant utilisation pour une parfaite mise en suspension.

Une telle préparation, à base de vésicules multilamellaires encapsulant l'actif présente les avantages de la rémanence de l'actif liée au phénomène d'adhésion et, de plus évite une pénétration systémique de l'actif dans l'épiderme grâce à la charge cationique des vésicules. Elle est donc particulièrement adaptée à tous les actifs qui ont une action locale sur le pelage des animaux.

### Exemple 7 : Formulation hydratante pour rouge à lèvres

L'introduction d'un hydratant hydrophile dans un rouge à lèvres, à base d'huile de ricin et de cire d'abeille, donc fortement hydrophobe pose d'insurmontables problèmes techniques. L'utilisation des vésicules multilamellaires selon l'invention, permet de résoudre ce problème, et apporte l'effet d'adhésion et donc de rémanence de l'hydratant sur les lèvres. Les vésicules sont préparées à partir de la formulation suivante :

| | |
|---|---|
| Tristéarate de sucrose | 30 % |
| Glycérol | 60% |
| Jaguar C13S | 1% |
| Eau | 9 % |

L'ensemble des constituants est grossièrement mélangé à température ambiante, puis la température est élevée jusqu'à 70°C, en maintenant une forte agitation. Lorsque le mélange est homogène, la température est abaissée lentement à l'ambiante en maintenant une agitation modérée. Le produit se présente sous la forme d'une pâte très ferme.

Cette pâte se disperse facilement dans l'huile de ricin, qui est ensuite utilisée pour préparer la base de rouge à lèvre, selon une formulation traditionnelle. On peut ainsi introduire jusqu'à 10% de vésicules dans le rouge à lèvres, correspondant à 6% de glycérol, sans dégrader les qualités cosmétiques du produit.

## Revendications

1. Procédé à l'exclusion d'un procédé de traitement thérapeutique du corps humain ou animal, destiné à faire adhérer un produit sur une surface, **caractérisé en ce qu'**il consiste à mettre en contact avec ladite surface une composition dans laquelle ledit produit est incorporé dans des vésicules multilamellaires sensiblement sphériques, de diamètre compris entre 0,1 et 100 µm, constituées de membranes concentriques à base d'au moins un tensioactif séparées par un milieu solvant, lesdites vésicules présentant une structure en oignon, et portant une charge globale positive liée à la présence d'au moins un agent cationique au sein desdites vésicules, ledit agent cationique étant un polymère cationique encapsulé dans lesdites vésicules multilamellaires.

2. Procédé selon la revendication 1, **caractérisé en ce que** ladite surface présente une charge négative.

3. Procédé selon l'une des revendications 1 ou 2, **caractérisé en ce que** ladite surface est constituée d'une fibre naturelle ou artificielle ou d'un ensemble de fibres tel qu'un tissu.

4. Procédé selon la revendication 1 ou 2, **caractérisé en ce que** ladite surface est une surface biologique, en particulier une partie externe du corps humain ou animal telle que la peau, les phanères, les poils ou les cheveux, ou la cuticule ou les pilosités des insectes ou une partie aérienne des végétaux, en particulier leur cuticule.

5. Procédé selon l'une des revendications 1 à 4, **caractérisé en ce que** lesdites membranes comprennent une quantité efficace d'au moins un tensioactif cationique pour conférer auxdites vésicules une charge positive.

6. Procédé selon la revendication 5, **caractérisé en ce que** ledit tensioactif cationique est choisi dans le groupe constitué des ammoniums quaternaires, des amines, des sels d'amine ou des amides susceptibles d'être protonés dans les conditions de pH d'utilisation de ladite composition, des dérivés de bétaïne ou d'aminoacides dans des conditions de pH qui les rendent cationiques, des dérivés d'imidazoline, des dialkylesters quaternisés.

7. Procédé selon la revendication 1, **caractérisé en ce que** ledit polymère cationique est présent en quantité efficace pour conférer auxdites vésicules une charge cationique.

8. Procédé selon la revendication 1, **caractérisé en ce que** ledit polymère cationique est choisi dans le groupe constitué des dérivés de polysaccharides, des hydrolysats cationiques de protéines, des dérivés polyaminés, des polyaminoacides dans les conditions de pH où ils sont cationiques, la polyéthylèneimine, les dérivés quaternaires de polyvinylpyrrolidone (PVP) et les copolymères de polyvinylpyrrolidone quatemisée et de polymères hydrophiles, les polyquatemium et les dérivés de la chitine.

9. Procédé selon l'une des revendications 1 à 8, **caractérisé en ce que** lesdites vésicules multilamellaires contiennent de 0,01 à 10 % en poids par rapport au poids desdites vésicules d'au moins un polymère cationique.

10. Utilisation des vésicules multilamellaires telles que définies dans l'une des revendications 1 ou 5 à 9, incorporant en leur sein un agent actif destiné au traitement topique de la peau ou de la chevelure des êtres humains ou de la peau ou du pelage des animaux, pour la fabrication d'une composition pharmaceutique ou vétérinaire destinée à faire adhérer ledit agent actif à ladite surface en vue du traitement topique de la peau ou de la chevelure des êtres humains ou de la peau ou du pelage des animaux.

11. Procédé selon l'une des revendications 1 à 9, **caractérisé en ce que** ladite composition mise en contact avec ladite surface est préparée selon un procédé comprenant les étapes de :
- préparation d'une phase lamellaire cristal-liquide à base d'au moins un agent tensioactif comprenant ledit polymère cationique et, le cas échéant, un produit à fixer ou un agent actif différent dudit polymère cationique,
- application d'un cisaillement de ladite phase lamellaire cristal-liquide conduisant à la formation desdites vésicules,
- incorporation desdites vésicules dans un milieu adapté.

## Claims

1. Method, excluding a therapeutic treatment method of human or animal body, intended to make a product adhere to a surface, **characterised in that** it consists in bringing into contact with said surface, a composition in which said product is incorporated in substantially spherical, multilamellar vesicles having a diameter of between 0.1 and 100 µm, consisting of concentric membranes based on at least one surfactant and separated by a solvent medium, said vesicles having an onion-like structure and carrying a positive overall charge due to the presence of at least one cationic agent encapsulated in said multilamellar vesicles, said cationic agent being a cationic polymer.

2. Method according to claim 1, **characterised in that** said surface has a negative charge.

3. Method according to any of claim 1 or 2, **characterised in that** said surface consists of a natural or artificial fiber or an assembly of fibers, such as a fabric.

4. Method according to claim 1 or 2, **characterised in that** said surface is a biological surface, particularly an external part of the human or animal body, such as the skin, integuments, body hair/fur, hair or the cuticle or hairs of insects, or an aerial part of plants, particularly their cuticle.

5. Method according to any one of claims 1 to 4, **characterised in that** said membranes comprise an effective amount of at least one cationic surfactant for imparting a positive charge to said vesicles.

6. Method according to claim 5, **characterised in that** said cationic surfactant is selected from the group consisting of quaternary ammonium compounds, amines, amine salts or amides which can be protonated under the pH conditions of use of said composition, betaine or amino acid derivatives under pH conditions which render them cationic, imidazoline derivatives and quaternised dialkyl esters.

7. Method according to claim 1, **characterised in that** said cationic polymer is present in an effective amount for imparting a cationic charge to said vesicles.

8. Method according to claim 1, **characterised in that** said cationic polymer is selected from the group consisting of polysaccharide derivatives, cationic protein hydrolysates, polyamine derivatives, polyamino acids under pH conditions where they are cationic, polyethylene imine, quaternary derivatives of polyvinylpyrrolidone (PVP) and copolymers of quaternised polyvinylpyrrolidone and hydrophilic polymers, polyquaternium compounds and chitin derivatives.

9. Method according to any one of claims 1 to 8, **characterised in that** said multilamellar vesicles contain from 0.01 to 10% by weight of at least one cationic polymer, based on the weight of said vesicles.

10. Use of multilamellar vesicles as claimed in any one of claims 1 or 5 to 9, incorporating an active agent intended for topical treatment of the skin, of the hair of humans or of the skin or fur of animals, for the manufacture of a pharmaceutical or veterinary composition intended to make said active agent adhere to said surface in view of the topical treatment of the skin or of the hair of humans or of the skin or fur of animals.

11. Method according to any one of claims 1 to 9, **characterised in that** said composition, brought into contact with said surface, is prepared using a method comprising the following steps:
- the preparation of a lamellar liquid crystal phase based on at least one surfactant comprising said cationic polymer and, if appropriate, a product to be fixed or an active agent other than said cationic polymer;
- the application of a shearing stress to said lamellar liquid crystal phase to form said vesicles;
- the incorporation of said vesicles into an appropriate medium.

## Patentansprüche

1. Verfahren, unter Ausschluss eines Verfahrens zur therapeutischen Behandlung des menschlichen oder tierischen Körpers, welches dazu bestimmt ist, ein Produkt auf einer Oberfläche haften zu lassen, **dadurch gekennzeichnet, dass** es darauf beruht, eine Zusammensetzung mit der Oberfläche in Kontakt zu bringen, bei der das Produkt in im Wesentlichen kugelförmige, multilamellare Vesikel mit einem Durchmesser zwischen 0,1 und 100 µm eingebettet ist, die von konzentrischen, durch ein Lösungsmittelmedium abgeschiedenen Membranen auf der Basis wenigstens eines Tensids gebildet sind, wobei die Vesikel eine Zwiebelstruktur aufweisen und eine positive Gesamtladung tragen, die mit dem Vorliegen wenigstens eines kationischen Mittels in den multilamellaren Vesikeln zusammenhängt, wobei das kationische Mittel ein kationisches Polymer ist.

2. Verfahren nach Anspruch 1, **dadurch gekennzeichnet, dass** die Oberfläche eine negative Ladung aufweist.

3. Verfahren nach einem der Ansprüche 1 oder 2, **dadurch gekennzeichnet, dass** die Oberfläche von einer natürlichen oder künstlichen Faser oder von einer Fasereinheit, wie beispielsweise einem Gewebe, gebildet ist.

4. Verfahren nach Anspruch 1 oder 2, **dadurch gekennzeichnet, dass** die Oberfläche eine biologische Oberfläche ist, insbesondere ein äußerer Teil des menschlichen oder tierischen Körpers, wie beispielsweise die Haut, die Anhangsgebilde der Haut, die Körper- oder Kopfhaare, oder die Kutikula oder die Behaarung von Insekten oder ein oberirdischer Teil von Pflanzen, insbesondere deren Kutikula.

5. Verfahren nach einem der Ansprüche 1 bis 4, **dadurch gekennzeichnet, dass** die Membranen eine wirkungsvolle Menge wenigstens eines kationischen Tensids enthalten, um den Vesikeln eine positive Ladung zu verleihen.

6. Verfahren nach Anspruch 5, **dadurch gekennzeichnet, dass** das kationische Tensid ausgewählt ist aus der Gruppe bestehend aus quaternärem Ammonium, Aminen, Aminsalzen oder Amiden, welche geeignet sind, unter den pH-Einsatzbedingungen der Zusammensetzung protoniert zu werden, Betain- oder Aminosäurederivaten unter pH-Bedingungen, die sie kationisch machen, Imidazolinderivaten, quaternisierten Dialkylestern.

7. Verfahren nach Anspruch 1, **dadurch gekennzeichnet, dass** das kationische Polymer in wirkungsvoller Menge vorhanden ist, um den Vesikeln eine kationische Ladung zu verleihen.

8. Verfahren nach Anspruch 1, **dadurch gekennzeichnet, dass** das kationische Polymer ausgewählt ist aus der Gruppe bestehend aus Polysaccharidderivaten, kationischen Proteinhydrolysaten, Polyaminderivaten, Polyaminosäuren unter pH-Bedingungen, unter denen sie kationisch sind, Polyethylenimin, quaternären Polyvinylpyrrolidonderivaten (PVP) und Copolymeren aus quaternisiertem Polyvinylpyrrolidon und hydrophilen Polymeren, Polyquaternium und Derivaten des Chitin.

9. Verfahren nach einem der Ansprüche 1 bis 8, **dadurch gekennzeichnet, dass** die multilamellaren Vesikel zwischen 0,01 und 10 Gew.-% bezogen auf das Gewicht der Vesikel wenigstens eines kationischen Polymers enthalten.

10. Verwendung der multilamellaren Vesikel wie sie in einem der Ansprüche 1 oder 5 bis 9 definiert sind, die einen Wirkstoff zur örtlichen Behandlung der Haut oder des Kopfhaars von Menschen oder der Haut oder des Haarkleids bzw. Fells von Tieren enthalten, für die Herstellung einer pharmazeutischen oder tierärztlichen Zusammensetzung, welche dazu bestimmt ist, den Wirkstoff im Hinblick auf die örtliche Behandlung der Haut oder der Kopfhaare von Menschen oder der Haut oder des Haarkleids bzw. Fells von Tieren auf der Oberfläche haften zu lassen.

11. Verfahren nach einem der Ansprüche 1 bis 9, **dadurch gekennzeichnet, dass** die mit der Oberfläche in Kontakt gebrachte Zusammensetzung nach einem Verfahren zubereitet wird, das die folgenden Schritte umfasst:
- Zubereitung einer lamellaren Flüssigkristallphase auf der Basis wenigstens eines Tensids enthaltend das kationische Polymer und gegebenenfalls ein Fixierungsmittel oder einen anderen Wirkstoff als das kationische Polymer,
- Durchführung einer Scherung der lamellaren Flüssigkristallphase, was zur Bildung der Vesikel führt,
- Einbetten der Vesikel in ein angepasstes Medium.
